# EUROPÄISCHE PATENTANMELDUNG

(11) **EP 0 623 611 A1**
(43) Veröffentlichungstag der Anmeldung: **09.11.1994**
(21) Anmeldenummer: 94106317.4
(22) Anmeldetag: 22.04.1994
(51) Int. Cl.: C07D 401/14, A61K 31/41

(54) **Pyridinylmethyl-substituierte Pyridine und Pyridone als Angiotensin II Antagonisten**

(30) Priorität: 06.05.1993 DE 4314964
(71) Anmelder: BAYER AG, 51368 Leverkusen (DE)
(72) Erfinder: Fey, Peter, Dr., D-42111 Wuppertal (DE); Dressel, Jürgen, Dr., D-42477 Radevormwald (DE); Hanko, Rudolf, Dr., D-45134 Essen (DE); Hübsch, Walter, Dr., D-42113 Wuppertal (DE); Krämer, Thomas, Dr., D-42111 Wuppertal (DE); Müller, Ulrich, Dr., D-42111 Wuppertal (DE); Müller-Gliemann, Matthias, Dr., D-42697 Solingen (DE); Beuck, Martin, Dr., D-40699 Erkrath (DE); Bischoff, Hilmar, Dr., D-42113 Wuppertal (DE); Wohlfeil, Stefan, Dr., D-40724 Hilden (DE); Denzer, Dirk, Dr., D-42115 Wuppertal (DE); Kazda, Stanislav, Prof. Dr., D-42115 Wuppertal (DE); Stasch, Johannes-Peter, Dr., D-42651 Solingen (DE); Knorr, Andreas, Dr., D-40699 Erkrath (DE); Zaiss, Siegfried, Dr., D-42113 Wuppertal (DE)

(57) **Zusammenfassung**

Pyridinylmethyl-substituierte Pyridine und Pyridone der allgemeinen Formel (I) 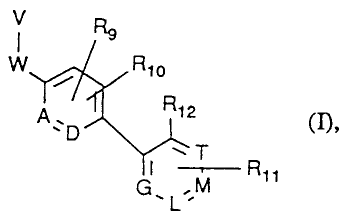
in welcher
A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
V für einen Rest der Formel 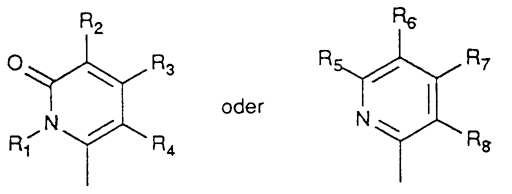
steht, werden hergestellt durch Umsetzen von Pyridin- bzw. Pyridon-substituierten Halogenpyridinen mit Tetrazolylphenylboronsäuren. Die erfindungsgemäßen Stoffe können als Wirkstoffe in Arzneimitteln, insbesondere zur Behandlung von arterieller Hypertonie und Atherosklerose verwendet werden.

## Beschreibung

Die Erfindung betrifft Pyridinylmethyl-substituierte Pyridine und Pyridone, Verfahren zu ihrer Herstellung sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Arylheteroarylalkyl substituierte Triazole und Imidazole sind aus den Publikationen EP 508 445, EP 503 393, EP 504 888 und US 5 128 327 als A II-Antagonisten bekannt.

Die vorliegende Erfindung betrifft Phenylpyridinylmethyl-substituierte 2-oxo-1,2-Dihydropyridine und Pyridine der allgemeinen Formel (I) 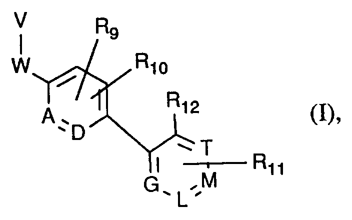
in welcher
- A, D, G, L, M und T: gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
- V: für einen Rest der Formel 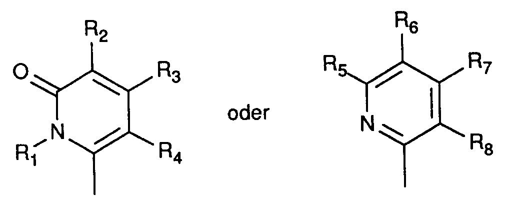 steht,
worin
- R¹: geradkettiges oder verzweigtes Alkyl mit bis zu 10 Kohlenstoffatomen bedeutet, das gegebenenfalls durch Cycloalkyl mit 3 bis 6 Kohlenstoffatomen, Hydroxy oder durch geradkettiges oder verzweigtes Alkoxy oder Alkylthio mit jeweils bis zu 6 Kohlenstoffatomen substituiert ist, oder
Cycloalkyl mit 3 bis 6 Kohlenstoffatomen bedeutet,
- R², R⁵ und R⁶: gleich oder verschieden sind und
Wasserstoff, Hydroxy, Nitro, Cyano, Formyl oder Halogen bedeuten, oder
geradkettiges oder verzweigtes Alkyl, Alkenyl, Alkinyl, Alkoxy oder Alkylthio mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder geradkettiges oder verzweigtes Acyl oder Alkoxycarbonyl mit jeweils bis zu 8 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 6 Kohlenstoffatomen substituiert ist, das seinerseits durch Cyano, Halogen, Carboxy, Phenoxycarbonyl, Hydroxy oder durch geradkettiges oder verzweigtes Alkoxy oder Alkoxycarbonyl mit jeweils bis zu 6 Kohlenstoffatomen substituiert sein kann, oder
eine Gruppe der Formel 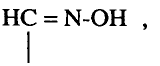 -NR¹³R¹⁴, -CO-NR¹⁵R¹⁶, -CH₂-OR¹⁷ oder -S(O)ₐ-R¹⁸ bedeuten
worin
- R¹³, R¹⁴, R¹⁵ und R¹⁶: gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
- R¹⁷: geradkettiges oder verzweigtes Acyl mit bis zu 6 Kohlenstoffatomen oder Benzoyl bedeutet,
- R¹⁸: geradkettiges oder verzweigtes Alkyl mit bis zu 8 Kohlenstoffatomen bedeutet,
- a: eine Zahl 1 oder 2 bedeutet,
- R³ und R⁷: gleich oder verschieden sind und
Wasserstoff, Hydroxy, Carboxy, geradkettiges oder verzweigtes Alkoxy oder Alkoxycarbonyl mit jeweils bis zu 8 Kohlenstoffatomen bedeuten, oder
eine Gruppe der Formel -NR¹⁹R²⁰ bedeuten,
worin
- R¹⁹ und R²⁰: die oben angegebene Bedeutung von R¹³ und R¹⁴ haben und mit dieser gleich oder verschieden sind,
oder
Aryl mit 6 bis 10 Kohlenstoffatomen bedeuten, das gegebenenfalls bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
geradkettiges oder verzweigtes Alkyl oder Alkenyl mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
- R⁴: Wasserstoff, Nitro, Carboxy oder geradkettiges oder verzweigtes Alkoxycarbonyl mit bis zu 8 Kohlenstoffatomen bedeutet, oder eine Gruppe der Formel -NR²¹R²² bedeutet,
worin
- R²¹ und R²²: die oben angegebene Bedeutung von R¹³ und R¹⁴ haben und mit dieser gleich oder verschieden sind,
oder
geradkettiges oder verzweigtes Alkyl oder Alkenyl mit jeweils bis zu 8 Kohlenstoffatomen bedeutet, die gegebenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
- R⁸: die oben angegebene Bedeutung von R¹ und R⁴ hat und mit diesen gleich oder verschieden ist,
- W: für die 〉C=O -Gruppe steht, oder
für eine Gruppe der Formel 〉CH-X steht,
worin
- X: Wasserstoff oder geradkettiges oder verzweigtes Alkyl mit bis zu 8 Kohlenstoffatomen bedeutet,
- R⁹, R¹⁰ und R¹¹: gleich oder verschieden sind und
für Wasserstoff, Halogen, Cyano, Nitro, Trifluormethyl, Hydroxy, Amido oder für geradkettiges oder verzweigtes Alkyl, Alkoxy oder Alkoxycarbonyl mit bis zu 6 Kohlenstoffatomen stehen,
- R¹²: für eine Gruppe der Formel -CO-R²³, -SO₂R²⁴, -CO-NR²⁵R²⁶, -NH-SO₂R²⁷ oder -SO₂NR²⁸R²⁹ steht,
worin
- R²³: Hydroxy oder geradkettiges oder verzweigtes Alkoxy mit bis zu 6 Kohlenstoffatomen bedeutet,
- R²⁴: Hydroxy, Trifluormethyl, geradkettiges oder verzweigtes Alkoxy oder Alkyl mit jeweils bis zu 6 Kohlenstoffatomen, Phenyl oder Benzyl bedeutet, die gegebenenfalls bis zu 2-fach gleich oder verschieden durch Halogen, Trifluormethyl oder geradkettiges oder verzweigtes Alkyl mit bis zu 4 Kohlenstoffatomen substituiert sind,
- R²⁵ und R²⁶: gleich oder verschieden sind und die oben angegebene Bedeutung von R¹³ und R¹⁴ haben,
oder
- R²⁵: Wasserstoff bedeutet
und
- R²⁶: die Gruppe -SO₂R²⁴ bedeutet,
worin
- R²⁴: die oben angegebene Bedeutung hat,
- R²⁷: die oben angegebene Bedeutung von R²⁴ hat und mit dieser gleich oder verschieden ist,
- R²⁸ und R²⁹: die oben angegebene Bedeutung von R¹³ und R¹⁴ haben und mit diesen gleich oder verschieden sind,
oder
- R²⁸: Wasserstoff oder geradkettiges oder verzweigtes Alkyl mit bis zu 4 Kohlenstoffatomen bedeutet
und
- R²⁹: die oben angegebene Bedeutung von R²⁴ hat und mit dieser gleich oder verschieden ist,
- R¹²: für einen Rest der Formel 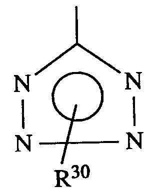 steht,
worin
- R³⁰: Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,
und deren Salze.

Die erfindungsgemäßen Phenylpyridinylmethyl-substituierte 2-oxo-1,2-dihydropyridine und Pyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Ein 5- bis 6-gliedriger, gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidyl. Bevorzugt ist Morpholinyl.

Bevorzugt sind Verbindungen der allgemeinen Formel (I),
in welcher
- A, D, G, L, M und T: gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
- V: für einen Rest der Formel 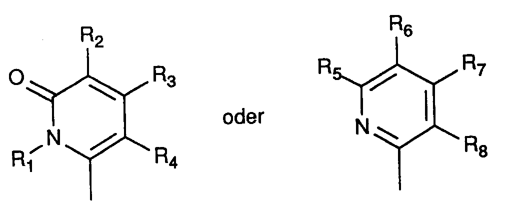 steht,
worin
- R¹: Wasserstoff, geradkettiges oder verzweigtes Alkyl mit jeweils bis zu 8 Kohlenstoffatomen bedeutet, das gegebenenfalls durch Cyclopropyl, Cyclopentyl, Cyclohexyl, Hydroxy oder durch geradkettiges oder verzweigtes Alkoxy oder Alkylthio mit jeweils bis zu 4 Kohlenstoffatomen substituiert ist, oder
Cyclopropyl, Cyclopentyl oder Cyclohexyl bedeutet,
- R², R⁵ und R⁶: gleich oder verschieden sind und
Wasserstoff, Hydroxy, Nitro, Cyano, Formyl, Fluor, Chlor, Brom oder Iod bedeuten, oder
geradkettiges oder verzweigtes Alkyl, Alkenyl, Alkinyl, Alkoxy oder Alkylthio mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 6 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 5 Kohlenstoffatomen substituiert ist, das seinerseits durch Cyano, Fluor, Chlor, Brom, Carboxy, Hydroxy oder durch geradkettiges oder verzweigtes Alkoxy oder Alkoxycarbonyl mit jeweils bis zu 4 Kohlenstoffatomen substituiert sein kann, oder

eine Gruppe der Formel 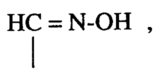 -NR¹³R¹⁴, -CO-NR¹⁵R¹⁶, -CH₂-OR¹⁷ oder -S(O)ₐ-R¹⁸ bedeuten,
worin
- R¹³, R¹⁴, R¹⁵ und R¹⁶: gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
- R¹⁷: geradkettiges oder verzweigtes Acyl mit bis zu 6 Kohlenstoffatomen oder Benzoyl bedeutet,
- R¹⁸: geradkettiges oder verzweigtes Alkyl mit bis zu 6 Kohlenstoffatomen bedeutet,
- a: eine Zahl 1 oder 2 bedeutet,
- R³ und R⁷: gleich oder verschieden sind und Wasserstoff, Hydroxy, Carboxy, geradkettiges oder verzweigtes Alkoxy oder Alkoxycarbonyl mit jeweils bis zu 6 Kohlenstoffatomen bedeuten, oder
eine Gruppe der Formel -NR¹⁹R²⁰ bedeuten,
worin
- R¹⁹ und R²⁰: die oben angegebene Bedeutung von R¹³ und R¹⁴ haben und mit dieser gleich oder verschieden sind,
oder
Phenyl bedeuten, das gegebenenfalls durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein kann, oder
geradkettiges oder verzweigtes Alkyl oder Alkenyl mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
- R⁴: Wasserstoff, Nitro, Carboxy oder geradkettiges oder verzweigtes Alkoxycarbonyl mit bis zu 6 Kohlenstoffatomen bedeutet, oder eine Gruppe der Formel -NR²¹R²² bedeutet,
worin
- R²¹ und R²²: die oben angegebene Bedeutung von R¹³ und R¹⁴ haben und mit dieser gleich oder verschieden sind,
oder
geradkettiges oder verzweigtes Alkyl oder Alkenyl mit jeweils bis zu 6 Kohlenstoffatomen bedeutet, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
- R⁴: die oben angegebene Bedeutung von R¹ und R⁸ hat und mit diesen gleich oder verschieden ist,
- W: für die 〉C=O -Gruppe steht, oder
für eine Gruppe der Formel 〉CH-X steht,
worin
- X: Wasserstoff oder geradkettiges oder verzweigtes Alkyl mit bis zu 7 Kohlenstoffatomen bedeutet,
- R⁹, R¹⁰ und R¹¹: gleich oder verschieden sind und
für Wasserstoff, Fluor, Chlor, Brom, Trifluormethyl, Hydroxy, Amido oder für geradkettiges oder verzweigtes Alkyl, Alkoxy oder Alkoxycarbonyl mit jeweils bis zu 4 Kohlenstoffatomen stehen,
- R¹²: für eine Gruppe der Formel -CO-R²³, -SO₂R²⁴, -CO-NR²⁵R²⁶, -NH-SO₂R²⁷ oder -SO₂NR²⁸R²⁹ steht,
worin
- R²³: Hydroxy oder geradkettiges oder verzweigtes Alkoxy mit bis zu 4 Kohlenstoffatomen bedeutet,
- R²⁴: geradkettiges oder verzweigtes Alkyl mit bis zu 4 Kohlenstoffatomen, Benzyl, Trifluormethyl oder p-Tolyl bedeutet,
- R²⁵ und R²⁶: gleich oder verschieden sind und die oben angegebene Bedeutung von R¹³ und R¹⁴ haben oder
- R²⁵: Wasserstoff bedeutet und
- R²⁶: die Gruppe -SO₂R²⁴ bedeutet,
worin
- R²⁴: die oben angegebene Bedeutung hat,
- R²⁷: die oben angegebene Bedeutung von R²⁴ hat und mit dieser gleich oder verschieden ist,
- R²⁸ und R²⁹: die oben angegebene Bedeutung von R¹³ und R¹⁴ haben und mit dieser gleich oder verschieden sind,
oder
- R²⁸: Wasserstoff oder Methyl bedeutet,
- R²⁹: die oben angegebene Bedeutung von R²⁴ hat und mit dieser gleich oder verschieden ist,
- R¹²: für einen Rest der Formel 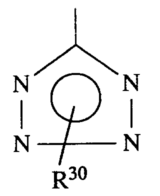 steht, worin
- R³⁰: Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

Besonders bevorzugt sind Verbindungen der allgemeinen Formel (I),
in welcher
- A, D, G, L, M und T: gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
- V: für einen Rest der Formel 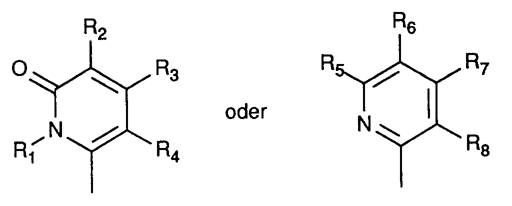 steht,
worin
- R¹: Wasserstoff oder geradkettiges oder verzweigtes Alkyl mit bis zu 6 Kohlenstoffatomen bedeutet, das gegebenenfalls durch Cyclopropyl, Hydroxy, geradkettiges oder verzweigtes Alkoxy oder Alkylthio mit jeweils bis zu 3 Kohlenstoffatomen substituiert ist oder Cyclopropyl, Chlor oder Iod bedeutet,
- R² und R⁶: gleich oder verschieden sind und Wasserstoff, Hydroxy, Nitro, Cyano, Formyl, Fluor, Chlor, Brom oder Iod bedeuten, oder
geradkettiges oder verzweigtes Alkyl, Alkenyl, Alkinyl, Alkoxy oder Alkylthio mit jeweils bis zu 4 Kohlenstoffatomen bedeuten, die gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert sind
geradkettiges oder verzweigtes Acyl oder Alkoxycarbonyl mit jeweils bis zu 4 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 4 Kohlenstoffatomen substituiert ist, das seinerseits durch Cyano, Fluor, Chlor, Brom, Carboxy, Hydroxy oder durch geradkettiges oder verzweigtes Alkoxy oder Alkoxycarbonyl mit jeweils bis zu 3 Kohlenstoffatomen substituiert sein kann, oder
eine Gruppe der Formel 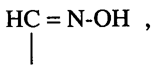 -NR¹³R¹⁴, -CO-NR¹⁵R¹⁶, -CH₂-OR¹⁷ oder -S(O)ₐ-R¹⁸ bedeuten,
worin
- R¹³, R¹⁴, R¹⁵ und R¹⁶: gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist,
- R¹⁷: geradkettiges oder verzweigtes Acyl mit bis zu 4 Kohlenstoffatomen oder Benzoyl bedeutet,
- R¹⁸: geradkettiges oder verzweigtes Alkyl mit bis zu 4 Kohlenstoffatomen bedeutet,
- a: eine Zahl 1 oder 2 bedeutet,
- R⁵: Hydroxy oder geradkettiges oder verzweigtes Alkyl mit bis zu 6 Kohlenstoffatomen bedeutet,
- R³ und R⁷: gleich oder verschieden sind und Wasserstoff, Hydroxy, Carboxy, geradkettiges oder verzweigtes Alkoxy oder Alkoxycarbonyl mit jeweils bis zu 4 Kohlenstoffatomen bedeuten, oder
eine Gruppe der Formel -NR¹⁹R²⁰ bedeuten,
worin
- R¹⁹ und R²⁰: die oben angegebene Bedeutung von R¹³ und R¹⁴ haben und mit dieser gleich oder verschieden sind,
oder
Phenyl bedeuten, das gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert ist, oder
Vinyl oder geradkettiges oder verzweigtes Alkyl mit bis zu 5 Kohlenstoffatomen bedeuten, das gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert ist,
- R⁴: Wasserstoff, Nitro, Carboxy oder geradkettiges oder verzweigtes Alkoxycarbonyl mit bis zu 4 Kohlenstoffatomen bedeutet, oder eine Gruppe der Formel -NR²¹R²² bedeutet,
worin
- R²¹ und R²²: die oben angegebene Bedeutung von R¹³ und R¹⁴ haben und mit dieser gleich oder verschieden sind,
oder
Vinyl oder geradkettiges oder verzweigtes Alkyl mit bis zu 4 Kohlenstoffatomen bedeutet, das gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert ist,
- R⁴: die oben angegebene Bedeutung von R¹ und R⁸ hat und mit dieser gleich oder verschieden ist,
- W: für die 〉C=O -Gruppe steht, oder
für eine Gruppe der Formel 〉CH-X steht,
worin
- X: Wasserstoff oder geradkettiges oder verzweigtes Alkyl mit bis zu 5 Kohlenstoffatomen bedeutet,
- R⁹, R¹⁰ und R¹¹: gleich oder verschieden sind und für Wasserstoff, Hydroxy, Fluor, Chlor, Brom oder Methyl stehen,
- R¹²: für eine Gruppe der Formel -CO-R²³, -SO₂-R²⁴, -CO-NR²⁵R²⁶, -NH-SO₂R²⁷ oder -SO₂-NR²⁸R²⁹ steht,
worin
- R²³: Hydroxy oder geradkettiges oder verzweigtes Alkoxy mit bis zu 3 Kohlenstoffatomen bedeutet,
- R²⁴: Methyl, Trifluormethyl, Benzyl oder p-Tolyl bedeutet,
- R²⁵ und R²⁶: gleich oder verschieden sind und die oben angegebene Bedeutung von R¹³ und R¹⁴ haben oder
- R²⁵: Wasserstoff bedeutet und
- R²⁶: die Gruppe -SO₂-R²⁴ bedeutet,
worin
- R²⁴: die oben angegebene Bedeutung hat,
- R²⁷: die oben angegebene Bedeutung von R²⁴ hat und mit dieser gleich oder verschieden ist,
- R²⁸ und R²⁹: die oben angegebene Bedeutung von R¹³ und R¹⁴ haben und mit dieser gleich oder verschieden sind,
oder
- R²⁸: Wasserstoff oder Methyl bedeutet
und
- R²⁹: die oben angegebene Bedeutung von R²⁴ hat und mit dieser gleich oder verschieden ist
- R¹²: für den Tetrazolylrest der Formel 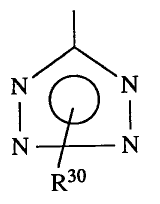 steht,
worin
- R³⁰: Wasserstoff oder geradkettiges oder verzweigtes Alkyl mit bis zu 4 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) 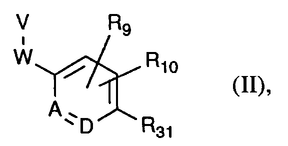
in welcher
- A, D, V, W, R⁹ und R¹⁰: die oben angegebene Bedeutung haben
und
- R³¹: für eine typische Abgangsgruppe, wie beispielsweise Brom, Jod, Methan-, Toluol-, Fluor- oder Trifluormethansulfonyloxy, vorzugsweise für Brom steht,
mit Verbindungen der allgemeinen Formel (III) oder (IIIa) 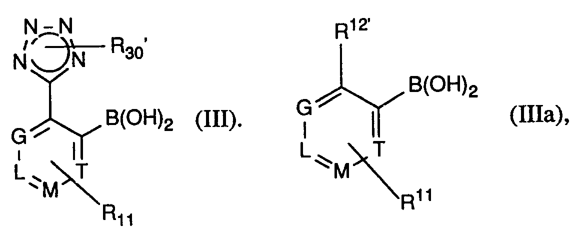
in welcher
- G, L, M, T und R¹¹: die oben angegebene Bedeutung haben,
- R^{30'}: für Wasserstoff oder für die Triphenylmethylgruppe steht,
und
- R^{12'}: die oben angegebene Bedeutung von R¹² hat, aber nicht für den Tetrazolylrest steht,
in inerten Lösemitteln, in Anwesenheit einer Base und metallkatalysiert umsetzt,
und anschließend im Fall R^{30'} = Triphenylmethylgruppe mit Säuren in organischen Lösemitteln und/oder Wasser nach üblichen Bedingungen abspaltet,
und gegebenenfalls im Fall der unter den Substituenten R¹² aufgeführten carbonylischen Reste nach Verseifung der jeweiligen Ester beispielsweise durch Amidierung oder Sulfoamidierung nach üblichen Methoden derivatisiert,
und im Fall der Salze bevorzugt ausgehend vom freien Tetrazol (R¹²/R^{30'} = H) mit Säuren oder Basen umsetzt,
und im Fall der freien Säure R¹² = CO₂H und des freien Tetrazols R³⁰ = H, ausgehend von den Disalzen mit Säuren umsetzt,
und gegebenenfalls auch die Substituenten R¹, R², R³, R⁴, R⁵, R⁶, R⁷, R⁸, R⁹, R¹⁰, R¹¹ und R¹² nach bekannten Methoden aufjeder Verfahrensstufe variiert.

Das erfindungsemäße Verfahren kann durch folgendes Formelschema beispielhaft erläutert werden: 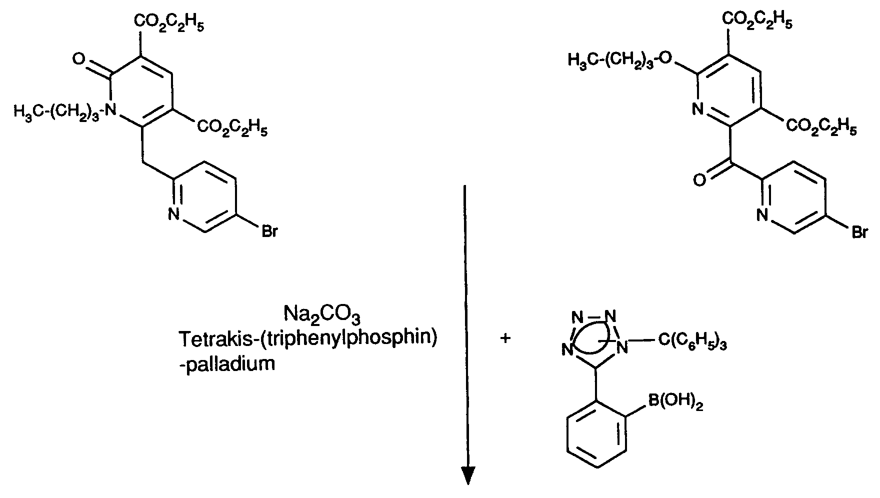 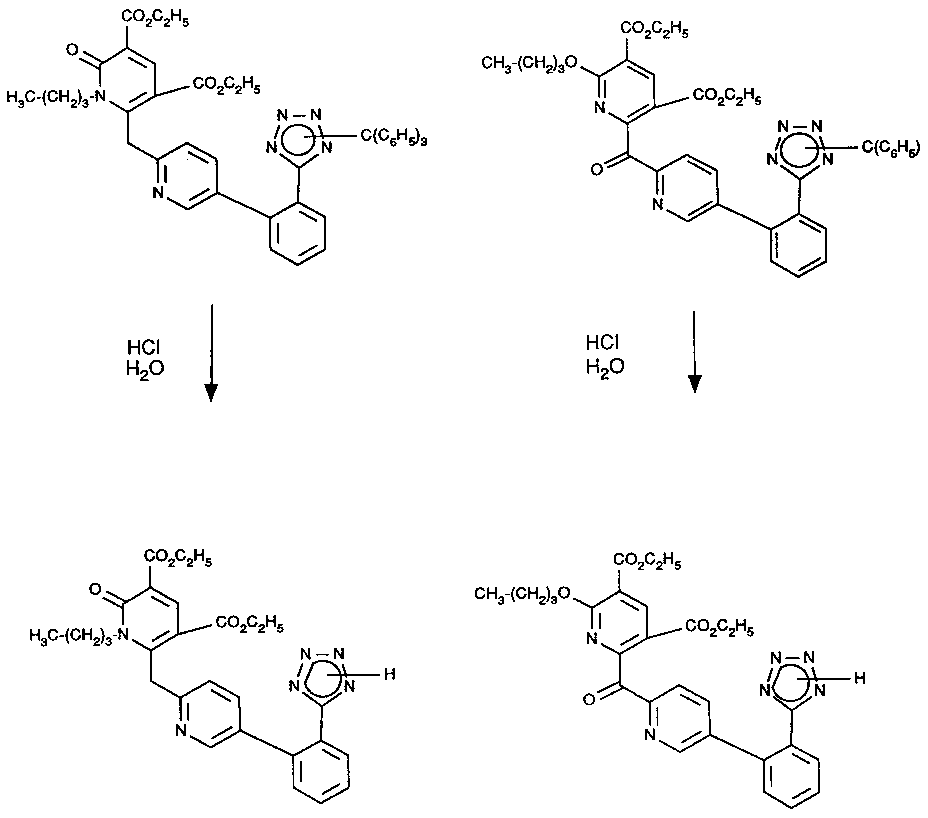
Als Lösemittel für das Verfahren eignen sich übliche organische Lösemittel, die sich unter den Reaktionsbedingungen nicht verändern. Hierzu gehören bevorzugt Wasser oder Alkohole, wie beispielsweise Methanol, Ethanol und Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel zu verwenden. Bevorzugt sind Tetrahydrofuran, Aceton, Dimethylformamid, Dimethoxyethan, Toluol und Methanol/Wasser.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkali- oder Erdalkalialkoholate oder -amide wie Natrium- oder Kaliummethanolat, Natrium- oder Kaliumethanolat oder Kalium-tert.butylat, Thalliumcarbonat oder -hydroxid, oder Lithiumdiisopropylamid (LDA), oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumhydrid, Kalium-tert.-butylat, Caesiumcarbonat, Natriumcarbonat, Thalliumhydroxid oder -carbonat.

Im allgemeinen setzt man die Base in einer Menge von 0,05 mol bis 10 mol, bevorzugt von 1 mol bis 2 mol, jeweils bezogen auf 1 mol der Verbindung der Formel (III), ein.

Die erfindungsgemäßen Verfahren werden im allgemeinen in einem Temperaturbereich von -100°C bis +100°C, bevorzugt von 0°C bis 80°C und Schutzgasatmosphäre durchgeführt.

Als Katalysatoren eignen sich im allgemeinen Metallkomplexe des Nickels, Palladiums oder Platins, bevorzugt Palladium(0)-Komplexe wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.
Außerdem eignen sich als Katalysatoren Kalium- oder Natriumiodid, bevorzugt Natriumiodid.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0°C bis 150°C, vorzugsweise von 20°C bis 100°C und Normaldruck.

Die Alkylierung erfolgt im allgemeinen mit Alkylierungsmitteln wie beispielsweise (C₁-C₆)-Alkylhalogeniden, Sulfonsäurestern oder substituierten oder unsubstituierten (C₁-C₆)-Dialkyl- oder (C₁-C₆)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20°C bis +80°C, vorzugsweise von -10°C bis +30°C und unter Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[4.3.0]-nonene-5 (DBN) oder 1,5-Diazabicyclo[5.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind größtenteils neu und können beispielsweise hergestellt werden, indem man
im Fall, daß V für den Rest der Formel 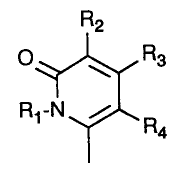
steht und W die 〉CH-X-Gruppe bedeutet,
Verbindungen der allgemeinen Formel (IV) 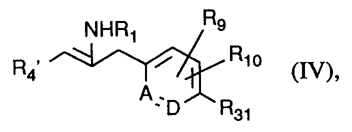
in welcher
- A, D, R¹, R⁹, R¹⁰ und R³¹: die oben angegebene Bedeutung haben
und
- R^{4'}: für Nitril oder für einen der oben unter R⁴ aufgeführten, chemisch sinnvollen Reste, bevorzugt für (C₁-C₄)-Alkoxycarbonyl, Nitro oder Nitril steht,
mit Verbindungen der allgemeinen Formel (V) 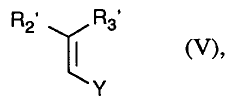
in welcher
- R^{2'} und R^{3'}: gleich oder verschieden sind und für Nitril oder für einen der oben unter R² und R³ aufgeführten, chemisch sinnvollen Reste, bevorzugt für (C₁-C₄)-Alkoxycarbonyl oder Nitril stehen,
und
- Y: für (C₁-C₄)-Alkoxy oder (C₁-C₄)-Dialkylamino steht,
umsetzt. Die Reduktion verläuft in einem Temperaturbereich von +50°C bis +130°C, vorzugsweise von +70°C bis +110°C und Normaldruck. Die Verbindungen der allgemeinen Formel (IV) sind teilweise bekannt oder neu und können dann beispielsweise hergestellt werden, indem man
Verbindungen der allgemeinen Formel (VI) 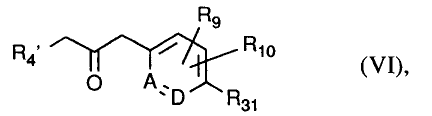
in welcher
- A, D, R^{4'}, R⁹, R¹⁰ und R³¹: die oben angegebene Bedeutung haben,
in einem der oben aufgeführten Lösemittel, vorzugsweise Ethanol, mit Ammoniak (R¹ = H) oder mit Aminen der allgemeinen Formel (VII)

H₂N-R¹ (VII),

in welcher
- R¹: die oben angegebene Bedeutung hat,
umsetzt.

Die Reaktion verläuft im allgemeinen in einem Temperaturbereich von -10°C bis +100°C, vorzugsweise von 0°C bis +100°C.

Die Amine der allgemeinen Formel (VII) sind bekannt.

Die Verbindungen der allgemeinen Formel (VI) sind teilweise bekannt oder neu und können dann beispielsweise hergestellt werden, indem man
Verbindungen der allgemeinen Formel (VIII) 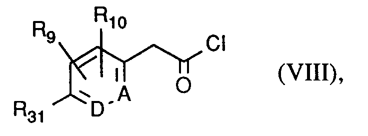
in welcher
- R⁹, R¹⁰ und R³¹: die oben angegebene Bedeutung haben,
mit Verbindungen der allgemeinen Formel (IX)

R^{4'}-CH₂-CO₂H (IX),

in welcher
- R^{4'}: die oben angegebene Bedeutung hat,
in einem der oben aufgeführten Lösemittel, vorzugsweise Tetrahydrofuran, umsetzt.

Die Umsetzung erfolgt in einem Temperaturbereich von 0°C bis +40°C, vorzugsweise bei +20°C.

Die Verbindungen der allgemeinen Formeln (VIII) und (IX) sind an sich bekannt.

Die Verbindungen der allgemeinen Formel (II), in welcher V für den Rest der Formel 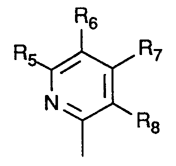
steht, sind ebenfalls neu und können beispielsweise hergestellt werden, indem man
Verbindungen der allgemeinen Formel (IIa) 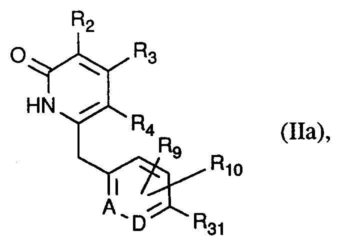
in welcher
- A, D, R², R³, R⁴, R⁹, R¹⁰ und R³¹: die oben angegebene Bedeutung haben,
in einem der oben aufgeführten Lösemittel, vorzugsweise Dimethylformamid, in Anwesenheit einer Base, vorzugsweise Caesiumcarbonat, zunächst mit Verbindungen der allgemeinen Formel (X)

R¹-Z (X),

in welcher
- R¹: die oben angegebene Bedeutung hat, aber vorzugsweise für (C₁-C₆)-Alkyl steht
und
- Z: für Halogen, vorzugsweise für Jod steht,
umsetzt.

Bei dieser Alkylierung entstehen außerdem die Verbindugen der allgemeinen Formel (II), in welcher W für die C=O-Gruppe steht.

Die Umsetzung erfolgt im allgemeinen in einem Temperaturbereich von 0°C bis +50°C, vorzugsweise von +10°C bis +30°C, und Normaldruck.

Die Base wird in einer Menge von 1 mol bis 10 mol, bevorzugt von 1 mol bis 3 mol, bezogen auf 1 mol der Verbindungen der allgemeinen Formel (X), eingesetzt.

Die Verbindungen der allgemeinen Formel (IIa) sind neu und können wie oben beschrieben hergestellt werden.

Die Verbindungen der allgemeinen Formel (X) sind bekannt.

Die Verbindungen der allgemeinen Formel (III) sind teilweise bekannt oder im Fall R³⁰ = H neu und können dann hergestellt werden, indem man Phenyltetrazol zunächst unter Schutzgasatmosphäre, in einem aprotischen Lösemittel und in Anwesenheit einer Base umsetzt, anschließend Borsäuretrimethylester zufügt und in einem letzten Schritt mit Säuren hydrolysiert.

Als Lösemittel eignen sich für das Verfahren aprotische Lösemittel wie Ether, beispielweise Tetrahydrofuran, Diethylether, Toluol, Hexan oder Benzol. Bevorzugt ist Tetrahydrofuran.

Als Basen eignen sich sec.- und tert.Butyllithium und Phenyllithium. Bevorzugt ist n-Butyllithium.

Die Base wird in einer Menge von 2 mol bis 5 mol, bevorzugt von 2 mol bis 3 mol, bezogen auf 1 mol Phenyltetrazol, eingesetzt.

Als Säuren eignen sich im allgemeinen Mineralsäuren, wie beispielsweise Salzsäure, C₁-C₄-Carbonsäuren, wie beispielsweise Essigsäure oder Phosphorsäure. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70°C bis +25°C, bevorzugt bei -10°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (IIIa) sind teilweise bekannt oder können nach üblichen Methoden hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen Phenylpyridinylmethyl-substituierten 2-oxo-1,2-Dihydropyridine und Pyridine zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Außerdem können die Verbindungen zur Bekämpfung von Glaukom, diabetischer Retinopathie Bewegungssteigerung der intraokularen Netzhautflüssigkeit eingesetzt werden.

Sie sind auch geeignet zur Bekämpfung von Erkrankungen des zentralen Nervensystems wie beispielsweise Depression, Migräne, Schizophrenie oder Angstzuständen, von Hirnleistungsstörungen, Schlaganfall, diabetischer Nephropathie, Herzrhytmusstörungen, Prophylaxe von koronaren Herzerkrankungen oder Restenoseprophylaxe nach Angioplastien und gefäßchrirugischen Maßnahmen.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert

### Agonisten und ihre Standardkonzentrationen (Applikationsvolumen pro Einzelgabe = 100 µl):

| | | |
|---|---|---|
| KCl | 22,7:32,7;42,7;52,7 | mmol/l |
| Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet.

Sobald die Blutdruckwerte ein stabiles Plateau erreicht haben, werden die Testsubstanzen entweder intravenös oder als Suspension bzw. Lösung in 0,5% Tylose oral verabreicht Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht
Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2), 5 mM MgCl₂ sowie die zu untersuchenden Substanzen enthält Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivitat korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert Danach werden die Zellen durch Serumentzug für 2 - 3 Tage synchronisiert und sodann mit Serum oder anderen Faktoren zum Wachstum angeregt Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fallen die genannte obere Grenze überschritten werden muß. Im Falle der Applikation größerer Mengen kann es empfehlenswert sein, diese in mehreren Einzelgaben über den Tag zu verteilen.

## Patentansprüche

1. Pyridinylmethyl-substituierte Pyridine und Pyridone der allgemeinen Formel (I) 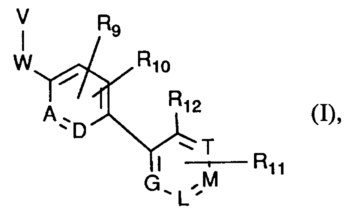 in welcher
A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
V für einen Rest der Formel 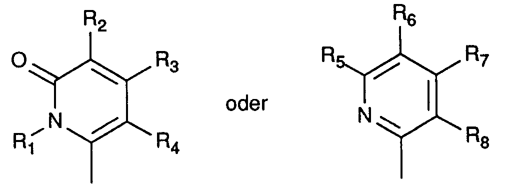 steht,
worin
R¹ geradkettiges oder verzweigtes Alkyl mit bis zu 10 Kohlenstoffatomen bedeutet, das gegebenenfalls durch Cycloalkyl mit 3 bis 6 Kohlenstoffatomen, Hydroxy oder durch geradkettiges oder verzweigtes Alkoxy oder Alkylthio mit jeweils bis zu 6 Kohlenstoffatomen substituiert ist, oder Cycloalkyl mit 3 bis 6 Kohlenstoffatomen bedeutet,
R², R⁵ und R⁶ gleich oder verschieden sind und
Wasserstoff, Hydroxy, Nitro, Cyano, Formyl oder Halogen bedeuten, oder
geradkettiges oder verzweigtes Alkyl, Alkenyl, Alkinyl, Alkoxy oder Alkylthio mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 8 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 6 Kohlenstoffatomen substituiert ist, das seinerseits durch Cyano, Halogen, Carboxy, Phenoxycarbonyl, Hydroxy oder durch geradkettiges oder verzweigtes Alkoxy oder Alkoxycarbonyl mit jeweils bis zu 6 Kohlenstoffatomen substituiert sein kann, oder
eine Gruppe der Formel 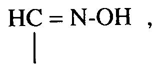 -NR¹³R¹⁴, -CO-NR¹⁵R¹⁶, -CH₂-OR¹⁷ oder -S(O)ₐ-R¹⁸ bedeuten
worin
R¹³, R¹⁴, R¹⁵ und R¹⁶ gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
R¹⁷ geradkettiges oder verzweigtes Acyl mit bis zu 6 Kohlenstoffatomen oder Benzoyl bedeutet,
R¹⁸ geradkettiges oder verzweigtes Alkyl mit bis zu 8 Kohlenstoffatomen bedeutet,
a eine Zahl 1 oder 2 bedeutet,
R³ und R⁷ gleich oder verschieden sind und
Wasserstoff, Hydroxy, Carboxy, geradkettiges oder verzweigtes Alkoxy oder Alkoxycarbonyl mit jeweils bis zu 8 Kohlenstoffatomen bedeuten, oder
eine Gruppe der Formel -NR¹⁹R²⁰ bedeuten,
worin
R¹⁹ und R²⁰ die oben angegebene Bedeutung von R¹³ und R¹⁴ haben und mit dieser gleich oder verschieden sind,
oder
Aryl mit 6 bis 10 Kohlenstoffatomen bedeuten, das gegebenenfalls bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
geradkettiges oder verzweigtes Alkyl oder Alkenyl mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
R⁴ Wasserstoff, Nitro, Carboxy oder geradkettiges oder verzweigtes Alkoxycarbonyl mit bis zu 8 Kohlenstoffatomen bedeutet, oder
eine Gruppe der Formel -NR²¹R²² bedeutet,
worin
R²¹ und R²² die oben angegebene Bedeutung von R¹³ und R¹⁴ haben und mit dieser gleich oder verschieden sind,
oder
geradkettiges oder verzweigtes Alkyl oder Alkenyl mit jeweils bis zu 8 Kohlenstoffatomen bedeutet, die gegebenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
R⁸ die oben angegebene Bedeutung von R¹ und R⁴ hat und mit diesen gleich oder verschieden ist,
W für die 〉C=O -Gruppe steht, oder
für eine Gruppe der Formel 〉CH-X steht,
worin
X Wasserstoff oder geradkettiges oder verzweigtes Alkyl mit bis zu 8 Kohlenstoffatomen bedeutet,
R⁹, R¹⁰ und R¹¹ gleich oder verschieden sind und
für Wasserstoff, Halogen, Cyano, Nitro, Trifluormethyl, Hydroxy, Amido oder für geradkettiges oder verzweigtes Alkyl, Alkoxy oder Alkoxycarbonyl mit bis zu 6 Kohlenstoffatomen stehen,
R¹² für eine Gruppe der Formel -CO-R²³, -SO₂R²⁴, -CO-NR²⁵R²⁶, -NH-SO₂R²⁷ oder -SO₂NR²⁸R²⁹ steht,
worin
R²³ Hydroxy oder geradkettiges oder verzweigtes Alkoxy mit bis zu 6 Kohlenstoffatomen bedeutet,
R²⁴ Hydroxy, Trifluormethyl, geradkettiges oder verzweigtes Alkoxy oder Alkyl mit jeweils bis zu 6 Kohlenstoffatomen, Phenyl oder Benzyl bedeutet, die gegebenenfalls bis zu 2-fach gleich oder verschieden durch Halogen, Trifluormethyl oder geradkettiges oder verzweigtes Alkyl mit bis zu 4 Kohlenstoffatomen substituiert sind,
R²⁵ und R²⁶ gleich oder verschieden sind und die oben angegebene Bedeutung von R¹³ und R¹⁴ haben,
oder
R²⁵ Wasserstoff bedeutet
und
R²⁶ die Gruppe -SO₂R²⁴ bedeutet,
worin
R²⁴ die oben angegebene Bedeutung hat,
R²⁷ die oben angegebene Bedeutung von R²⁴ hat und mit dieser gleich oder verschieden ist,
R²⁸ und R²⁹ die oben angegebene Bedeutung von R¹³ und R¹⁴ haben und mit diesen gleich oder verschieden sind,
oder
R²⁸ Wasserstoff oder geradkettiges oder verzweigtes Alkyl mit bis zu 4 Kohlenstoffatomen bedeutet
und
R²⁹ die oben angegebene Bedeutung von R²⁴ hat und mit dieser gleich oder verschieden ist,
R¹² für einen Rest der Formel 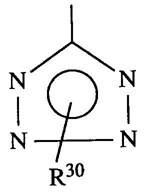 steht,
worin
R³⁰ Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,
und deren Salze.

2. Pyridinylmethyl-substituierte Pyridine und Pyridone nach Anspruch 1,
worin
A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
V für einen Rest der Formel 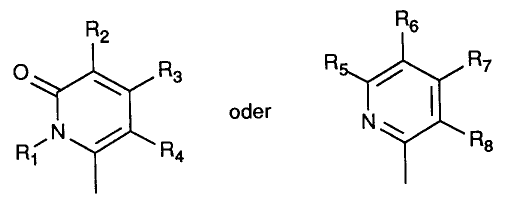 steht,
worin
R¹ Wasserstoff, geradkettiges oder verzweigtes Alkyl mit jeweils bis zu 8 Kohlenstoffatomen bedeutet, das gegebenenfalls durch Cyclopropyl, Cyclopentyl, Cyclohexyl, Hydroxy oder durch geradkettiges oder verzweigtes Alkoxy oder Alkylthio mit jeweils bis zu 4 Kohlenstoffatomen substituiert ist, oder Cyclopropyl, Cyclopentyl oder Cyclohexyl bedeutet,
R², R⁵ und R⁶ gleich oder verschieden sind und
Wasserstoff, Hydroxy, Nitro, Cyano, Formyl, Fluor, Chlor, Brom oder Iod bedeuten, oder
geradkettiges oder verzweigtes Alkyl, Alkenyl, Alkinyl, Alkoxy oder Alkylthio mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 6 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 5 Kohlenstoffatomen substituiert ist, das seinerseits durch Cyano, Fluor, Chlor, Brom, Carboxy, Hydroxy oder durch geradkettiges oder verzweigtes Alkoxy oder Alkoxycarbonyl mit jeweils bis zu 4 Kohlenstoffatomen substituiert sein kann, oder
eine Gruppe der Formel 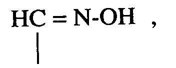 -NR¹³R¹⁴, -CO-NR¹⁵R¹⁶, -CH₂-OR¹⁷ oder -S(O)ₐ-R¹⁸ bedeuten,
worin
R¹³, R¹⁴, R¹⁵ und R¹⁶ gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
R¹⁷ geradkettiges oder verzweigtes Acyl mit bis zu 6 Kohlenstoffatomen oder Benzoyl bedeutet,
R¹⁸ geradkettiges oder verzweigtes Alkyl mit bis zu 6 Kohlenstoffatomen bedeutet,
a eine Zahl 1 oder 2 bedeutet,
R³ und R⁷ gleich oder verschieden sind und Wasserstoff, Hydroxy, Carboxy, geradkettiges oder verzweigtes Alkoxy oder Alkoxycarbonyl mit jewiels bis zu 6 Kohlenstoffatomen bedeuten, oder
eine Gruppe der Formel -NR¹⁹R²⁰ bedeuten,
worin
R¹⁹ und R²⁰ die oben angegebene Bedeutung von R¹³ und R¹⁴ haben und mit dieser gleich oder verschieden sind,
oder
Phenyl bedeuten, das gegebenenfalls durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein kann, oder
geradkettiges oder verzweigtes Alkyl oder Alkenyl mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
R⁴ Wasserstoff, Nitro, Carboxy oder geradkettiges oder verzweigtes Alkoxycarbonyl mit bis zu 6 Kohlenstoffatomen bedeutet, oder
eine Gruppe der Formel -NR²¹R²² bedeutet,
worin
R²¹ und R²² die oben angegebene Bedeutung von R¹³ und R¹⁴ haben und mit dieser gleich oder verschieden sind,
oder geradkettiges oder verzweigtes Alkyl oder Alkenyl mit jeweils bis zu 6 Kohlenstoffatomen bedeutet, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
R⁴ die oben angegebene Bedeutung von R¹ und R⁸ hat und mit diesen gleich oder verschieden ist,
W für die 〉C=O -Gruppe steht, oder
für eine Gruppe der Formel 〉CH-X steht,
worin
X Wasserstoff oder geradkettiges oder verzweigtes Alkyl mit bis zu 7 Kohlenstoffatomen bedeutet,
R⁹, R¹⁰ und R¹¹ gleich oder verschieden sind und
für Wasserstoff, Fluor, Chlor, Brom, Trifluormethyl, Hydroxy, Amido oder für geradkettiges oder verzweigtes Alkyl, Alkoxy oder Alkoxycarbonyl mit jeweils bis zu 4 Kohlenstoffatomen stehen,
R¹² für eine Gruppe der Formel -CO-R²³, -SO₂R²⁴, -CO-NR²⁵R²⁶, -NH-SO₂R²⁷ oder -SO₂NR²⁸R²⁹ steht,
worin
R²³ Hydroxy oder geradkettiges oder verzweigtes Alkoxy mit bis zu 4 Kohlenstoffatomen bedeutet,
R²⁴ geradkettiges oder verzweigtes Alkyl mit bis zu 4 Kohlenstoffatomen, Benzyl, Trifluormethyl oder p-Tolyl bedeutet,
R²⁵ und R²⁶ gleich oder verschieden sind und die oben angegebene Bedeutung von R¹³ und R¹⁴ haben oder
R²⁵ Wasserstoff bedeutet und
R²⁶ die Gruppe -SO₂R²⁴ bedeutet,
worin
R²⁴ die oben angegebene Bedeutung hat,
R²⁷ die oben angegebene Bedeutung von R²⁴ hat und mit dieser gleich oder verschieden ist,
R²⁸ und R²⁹ die oben angegebene Bedeutung von R¹³ und R¹⁴ haben und mit dieser gleich oder verschieden sind,
oder
R²⁸ Wasserstoff oder Methyl bedeutet,
R²⁹ die oben angegebene Bedeutung von R²⁴ hat und mit dieser gleich oder verschieden ist,
R¹² für einen Rest der Formel 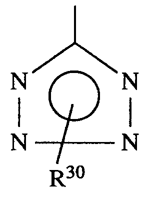 steht,
worin
R³⁰ Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

3. Pyridinylmethyl-substituierte Pyridine und Pyridone nach Anspruch 1, worin
A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,
V für einen Rest der Formel 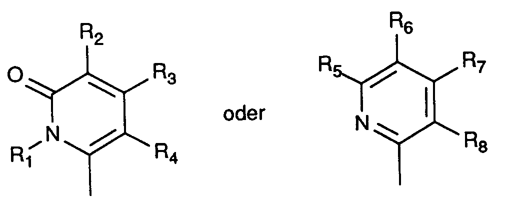 steht,
worin
R¹ Wasserstoff oder geradkettiges oder verzweigtes Alkyl mit bis zu 6 Kohlenstoffatomen bedeutet, das gegebenenfalls durch Cyclopropyl, Hydroxy, geradkettiges oder verzweigtes Alkoxy oder Alkylthio mit jeweils bis zu 3 Kohlenstoffatomen substituiert ist oder
Cyclopropyl, Chlor oder Iod bedeutet,
R² und R⁶ gleich oder verschieden sind und Wasserstoff, Hydroxy, Nitro, Cyano, Formyl, Fluor, Chlor, Brom oder Iod bedeuten, oder
geradkettiges oder verzweigtes Alkyl, Alkenyl, Alkinyl, Alkoxy oder Alkylthio mit jeweils bis zu 4 Kohlenstoffatomen bedeuten, die gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert sind
geradkettiges oder verzweigtes Acyl oder Alkoxycarbonyl mit jeweils bis zu 4 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 4 Kohlenstoffatomen substituiert ist, das seinerseits durch Cyano, Fluor, Chlor, Brom, Carboxy, Hydroxy oder durch geradkettiges oder verzweigtes Alkoxy oder Alkoxycarbonyl mit jeweils bis zu 3 Kohlenstoffatomen substituiert sein kann, oder
eine Gruppe der Formel 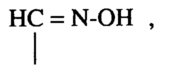 -NR¹³R¹⁴, -CO-NR¹⁵R¹⁶, -CH₂-OR¹⁷ oder -S(O)ₐ-R¹⁸ bedeuten,
worin
R¹³, R¹⁴, R¹⁵ und R¹⁶ gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist,
R¹⁷ geradkettiges oder verzweigtes Acyl mit bis zu 4 Kohlenstoffatomen oder Benzoyl bedeutet,
R¹⁸ geradkettiges oder verzweigtes Alkyl mit bis zu 4 Kohlenstoffatomen bedeutet,
a eine Zahl 1 oder 2 bedeutet,
R⁵ Hydroxy oder geradkettiges oder verzweigtes Alkyl mit bis zu 6 Kohlenstoffatomen bedeutet,
R³ und R⁷ gleich oder verschieden sind und Wasserstoff, Hydroxy, Carboxy, geradkettiges oder verzweigtes Alkoxy oder Alkoxycarbonyl mit jeweils bis zu 4 Kohlenstoffatomen bedeuten, oder
eine Gruppe der Formel -NR¹⁹R²⁰ bedeuten,
worin
R¹⁹ und R²⁰ die oben angegebene Bedeutung von R¹³ und R¹⁴ haben und mit dieser gleich oder verschieden sind,
oder
Phenyl bedeuten, das gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert ist, oder
Vinyl oder geradkettiges oder verzweigtes Alkyl mit bis zu 5 Kohlenstoffatomen bedeuten, das gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert ist,
R⁴ Wasserstoff, Nitro, Carboxy oder geradkettiges oder verzweigtes Alkoxycarbonyl mit bis zu 4 Kohlenstoffatomen bedeutet, oder
eine Gruppe der Formel -NR²¹R²² bedeutet,
worin
R²¹ und R²² die oben angegebene Bedeutung von R¹³ und R¹⁴ haben und mit dieser gleich oder verschieden sind,
oder
Vinyl oder geradkettiges oder verzweigtes Alkyl mit bis zu 4 Kohlenstoffatomen bedeutet, das gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert ist,
R⁴ die oben angegebene Bedeutung von R¹ und R⁸ hat und mit dieser gleich oder verschieden ist,
W für die 〉C=O -Gruppe steht, oder
für eine Gruppe der Formel 〉CH-X steht,
worin
X Wasserstoff oder geradkettiges oder verzweigtes Alkyl mit bis zu 5 Kohlenstoffatomen bedeutet,
R⁹, R¹⁰ und R¹¹ gleich oder verschieden sind und
für Wasserstoff, Hydroxy, Fluor, Chlor, Brom oder Methyl stehen,
R¹² für eine Gruppe der Formel -CO-R²³, -SO₂-R²⁴, -CO-NR²⁵R²⁶, -NH-SO₂R²⁷ oder -SO₂-NR²⁸R²⁹ steht,
worin
R²³ Hydroxy oder geradkettiges oder verzweigtes Alkoxy mit bis zu 3 Kohlenstoffatomen bedeutet,
R²⁴ Methyl, Trifluormethyl, Benzyl oder p-Tolyl bedeutet,
R²⁵ und R²⁶ gleich oder verschieden sind und die oben angegebene Bedeutung von R¹³ und R¹⁴ haben oder
R²⁵ Wasserstoff bedeutet und
R²⁶ die Gruppe -SO₂-R²⁴ bedeutet,
worin
R²⁴ die oben angegebene Bedeutung hat,
R²⁷ die oben angegebene Bedeutung von R²⁴ hat und mit dieser gleich oder verschieden ist,
R²⁸ und R²⁹ die oben angegebene Bedeutung von R¹³ und R¹⁴ haben und mit dieser gleich oder verschieden sind,
oder
R²⁸ Wasserstoff oder Methyl bedeutet
und
R²⁹ die oben angegebene Bedeutung von R²⁴ hat und mit dieser gleich oder verschieden ist
R¹² für den Tetrazolylrest der Formel 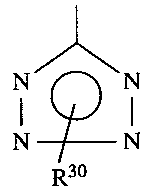 steht,
worin
R³⁰ Wasserstoff oder geradkettiges oder verzweigtes Alkyl mit bis zu 4 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

4. Pyridinylmethyl-substituierte Pyridine und Pyridone zur therapeutischen Anwendung.

5. Verfahren zur Herstellung von Pyridinylmethyl-substituierten Pyridinen und Pyridonen nach Ansprüchen 1 bis 3,
dadurch gekennzeichnet, daß man
Verbindungen der allgemeinen Formel (II) 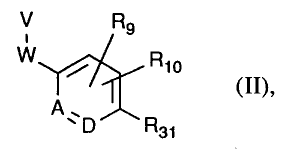 in welcher
A, D, V, W, R⁹ und R¹⁰ die oben angegebene Bedeutung haben
und
R³¹ für eine typische Abgangsgruppe, wie beispielsweise Brom, Jod, Methan-, Toluol-, Fluor- oder Trifluormethansulfonyloxy, vorzugsweise für Brom steht,
mit Verbindungen der allgemeinen Formel (III) oder (IIIa) 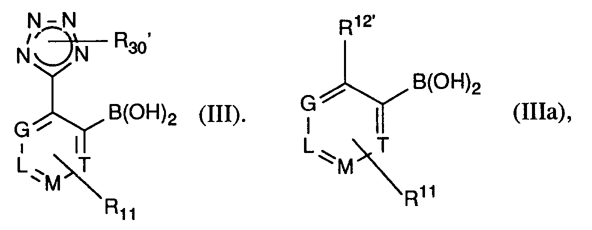 in welcher
G, L, M, T und R¹¹ die oben angegebene Bedeutung haben,
R^{30'} für Wasserstoff oder für die Triphenylmethylgruppe steht,
und
R^{12'} die oben angegebene Bedeutung von R¹² hat, aber nicht für den Tetrazolylrest steht,
in inerten Lösemitteln, in Anwesenheit einer Base und metallkatalysiert umsetzt,
und anschließend im Fall R^{30'} = Triphenylmethylgruppe mit Säuren in organischen Lösemitteln und/oder Wasser nach üblichen Bedingungen abspaltet,
und gegebenenfalls im Fall der unter den Substituenten R¹² aufgeführten carbonylischen Reste nach Verseifung der jeweiligen Ester beispielsweise durch Amidierung oder Sulfoamidierung nach üblichen Methoden derivatisiert,
und im Fall der Salze bevorzugt ausgehend vom freien Tetrazol (R¹²/R^{30'} = H) mit Säuren oder Basen umsetzt,
und im Fall der freien Säure R¹² = CO₂H und des freien Tetrazols R³⁰ = H, ausgehend von den Disalzen mit Säuren umsetzt,
und gegebenenfalls auch die Substituenten R¹, R², R³, R⁴, R⁵, R⁶, R⁷, R⁸, R⁹, R¹⁰, R¹¹ und R¹² nach bekannten Methoden auf jeder Verfahrensstufe variiert.

6. Arzneimittel enthaltend mindestens ein Pyridinylmethyl-substituiertes Pyridin oder Pyridon nach Ansprüchen 1 bis 3.

7. Arzneimittel nach Anspruch 6 zur Behandlung von arterieller Hypertonie und Atherosklerose.

8. Verfahren zur Herstellung von Arzneimitteln nach Anspruch 6 und 7, dadurch gekennzeichnet, daß man die Pyridinylmethyl-substituierten Pyridine oder Pyridone, gegebenenfalls mit Hilfe von geeigneten Hilfs- und Trägerstoffen, in eine geeignete Applikationsform überführt.

9. Verwendung von Pyridinylmethyl-substituierten Pyridinen und Pyridonen nach Anspruch 1 bis 3 zur Herstellung von Arzneimitteln.

10. Verwendung nach Anspruch 9 zur Herstellung von Arzneimitteln zur Behandlung von arterieller Hypertonie und Atherosklerose.
